# EUROPEAN PATENT APPLICATION

(11) **EP 1 767 184 A1**
(43) Date of publication of application: **28.03.2007**
(21) Application number: 06019281.2
(22) Date of filing: 14.09.2006
(51) Int. Cl.: A61K 6/083

(54) **Dental paste glass ionomer cement composition**

(30) Priority: 27.09.2005 JP 2005280327
(71) Applicant: GC Corporation, Tokyo 174-8585 (JP)
(72) Inventor: Noguchi, Tsuyoshi GC Corp., Itabashi-ku Tokyo 174-8585 (JP); Kato, Katsuhito GC Corp., Itabashi-ku Tokyo 174-8585 (JP); Nakaseko, Hisashi GC Corp., Itabashi-ku Tokyo 174-8585 (JP)
(74) Representative: Müller-Boré & Partner Patentanwälte

(57) **Abstract**

Such a dental paste glass ionomer cement composition is provided that has the similar operationality as a conventional dental glass ionomer cement, is high in mechanical strength, particularly compression strength, and is excellent in operationality of a two-paste system. The dental paste glass ionomer cement composition comprises a first paste comprising an α-β unsaturated carboxylic acid polymer, a filler that is not reacted with the α-β unsaturated carboxylic acid polymer and is not in a monodisperse state in water, a silica aqueous sol containing colloidal silica having an average particle diameter of 1 to 100 nm dispersed in a monodisperse state in water to an SiO₂ concentration of 1 to 50% by weight, and water, in prescribed amounts respectively, and a second paste containing fluoroalumino silicate glass powder in a prescribed amount. The second paste sometimes contains a thickening agent comprising a water soluble polymer material and water, or a polymerizable monomer having no acid group.

## Description

The present invention relates to a dental paste glass ionomer cement composition that is particularly improved in compression strength among physical strength of a hardened body thereof.

A dental glass ionomer cement is a dental cement used by hardening fluoroalumino silicate powder and an α-β unsaturated carboxylic acid polymer through reaction in the presence of water, and is being widely used owing to various excellent characteristics, such as considerably good affinity to a living body, excellent esthetic property with a translucent hardened body, excellent adhesion property to tooth substance, e.g., enamel and dentin, and cariostatic function of fluorine contained in fluoroalumino silicate glass powder as glass powder for the glass ionomer cement.

The dental glass ionomer cement having various excellent characteristics is being applied to wide variation of purposes in the dental field, such as restoration of a caries cavity, cementation of a crown, an inlay, a bridge or an orthodontic band, lining of a cavity, sealing for restoring a root canal, core construction, and preventive sealing.

The dental glass ionomer cement is ordinarily in such a formulation that fluoroalumino silicate powder and an aqueous solution of an α-β unsaturated carboxylic acid polymer are mixed and kneaded immediately before use, and in order to improve the physical strength of a hardened body of the dental glass ionomer cement, particularly the flexural strength thereof, a resin-reinforced dental glass ionomer cement is being developed, in which a polymerizable monomer is added to an aqueous solution of an α-β unsaturated carboxylic acid polymer.

In recent years, furthermore, a paste type dental glass ionomer cement is proposed, in which a first paste obtained by kneading a polymerizable monomer and fluoroalumino silicate glass powder, and a second paste containing an aqueous solution of an α-β unsaturated carboxylic acid polymer as a major component are mixed and kneaded immediately before use (as described in JP-A-11-228327, patent document 1).

A hardened body of the conventional dental glass ionomer cement sometimes suffers, upon application of stress thereto, breakage due to cracks formed from minute pores and defects inside the hardened body and flaws on the surface of the hardened body. It is considered that this is because, as compared to a glass part having a uniform three-dimensional network structure constituted by firm covalent bonds of Si-O or Al-O, the matrix part constituted by reaction of the α-β unsaturated carboxylic acid polymer, water and the surface of the glass is brittle, and upon concentrating stress to a minute crack formed in a part of the hardened body, the crack rapidly proceeds in the matrix part having a low strength with avoiding the glass part having a high strength, so as to bring about breakage of the hardened body. Therefore, in order to attain excellent mechanical property in a hardened body of the dental glass ionomer cement, it is effective to reduce the amount of the matrix part by increasing the content of the glass powder, and in other words, to increase the breakage proceeding distance to disperse the stress by increasing the charging amount of the powder component. However, if the powder and the liquid are mixed and kneaded with an increased charging amount of the powder, it is difficult to mix them well, and such a problem occurs in operationality as a dental cement that the mixed and kneaded paste has poor flowability as a dental material.

In order to improve the physical strength without impairing the operationality as a dental material, there has been proposed such a high-strength calcium phosphate cement that is obtained by mixing powder and water with an optimized mixing ratio of α-type calcium tertiary phosphate, calcium quaternary phosphate and calcium secondary phosphate (as described, for example, in JP-A-6-172007, patent document 2). However, it cannot have a sufficient strength as a dental material since a calcium phosphate cement inherently has a low physical strength.

Such dental cements are also proposed that are improved in mechanical strength by incorporating fibrous chips having a high strength or CPSA glass fiber fine powder (as described, for example, in JP-A-59-161307 and JP-A-2000-119119, patent documents 3 and 4). However, when using these fibrous fillers, there are some cases where directional dependency occurs in improvement of the strength, which brings about such a problem that the compression strength is substantially not improved although the flexural strength is improved. Furthermore, incorporation of the fibrous fillers brings about such practical problems that the cement is hard to be mixed, and the surface smoothness and glossiness of the hardened body are lost when the ends of the fibrous fillers are exposed on the surface due to abrasion or the like.

An object of the present invention is to provide such a dental paste glass ionomer cement composition that has the similar operationality to a conventional dental glass ionomer cement, is high in mechanical strength, particularly compression strength, and is excellent in operationality of a two-paste system.

As a result of earnest investigations made by the inventors to solve the problems associated with the conventional techniques, the aforementioned objects of the present invention can be attained by mixing a prescribed amount in terms of colloidal silica amount of a silica aqueous sol containing colloidal silica having an average particle diameter of 1 to 100 nm dispersed in a monodisperse state in water to an SiO₂ concentration of 1 to 50% by weight, with a first paste containing a prescribed amount of an α-β unsaturated carboxylic acid polymer, a prescribed amount of a f iller that is not reacted with the α-β unsaturated carboxylic acid polymer and is not in a monodisperse state in water, and water.

The present invention relates to a dental paste glass ionomer cement composition comprising:
a first paste comprising:
   20 to 60% byweight of an α-β unsaturated carboxylic acid polymer,
   10 to 60% by weight of a filler that is not reacted with the α-β unsaturated carboxylic acid polymer and is not in a monodisperse state in water,
   0.1 to 10% by weight in terms of colloidal silica amount of a silica aqueous sol containing colloidal silica having an average particle diameter of 1 to 100 nm dispersed in a monodisperse state in water to an SiO₂ concentration of 1 to 50% by weight, and
   20 to 60% by weight as the balance of water,
   and
a second paste that is reacted with the first paste by mixing therewith at a prescribed mixing ratio,
the second paste comprising:
   50 to 85% byweight of fluoroalumino silicate glass powder,
   0.01 to 10% by weight of a thickening agent comprising a water soluble polymer material, and
   20 to 45% by weight as the balance of water.

It is preferred in the dental paste glass ionomer cement composition according to the present invention that the second paste comprises:
50 to 85% by weight of fluoroalumino silicate glass powder,
0.01 to 10% by weight of a thickening agent comprising a water soluble polymer material,
0.1 to 10% by weight in terms of colloidal silica amount of a silica aqueous sol containing colloidal silica having an average particle diameter of 1 to 100 nm dispersed in a monodisperse state in water to an SiO₂ concentration of 1 to 50% by weight, and
20 to 45% by weight as the balance of water.

In this preferred embodiment, the amount of the monodisperse colloidal silica contained in the total dental paste glass ionomer cement composition is increased, whereby the mechanical strength, particularly the compression strength, is favorably improved.

It is also preferred in the dental paste glass ionomer cement composition according to the present invention that the second paste comprises:
50 to 85% by weight of fluoroalumino silicate glass powder, and
15 to 50% by weight of a polymerizable monomer having no acid group, and
at least one of the second paste and the first paste comprises a polymerization catalyst in an amount of 0.05 to 10 parts by weight per 100 parts by weight of a mixture containing the first paste and the second paste in a prescribed mixing ratio.

In this preferred embodiment, the mechanical strength, particularly the compression strength, of a resin-reinforced dental glass ionomer cement, which contains a polymerizable monomer for improving the mechanical strength, particularly the flexural strength, of a hardened body thereof, is favorably improved.

It is also preferred in the dental paste glass ionomer cement composition according to the present invention that the α-β unsaturated carboxylic acid polymer is a copolymer or a homopolymer containing at least one selected from acrylic acid, methacrylic acid, 2-chloroacrylic acid, 3-chloroacrylic acid, aconitic acid, mesaconic acid, maleic acid, itaconic acid, fumaric acid, glutaconic acid and citraconic acid, containing no polymerizable ethylenic unsaturated double bond, and having a weight average molecular weight of 5,000 to 40,000. It is also preferred that the filler that is not reacted with the α-β unsaturated carboxylic acid polymer and is not in a monodisperse state in water is at least one selected from SiO₂, Al₂O₃ and TiO₂. It is also preferred that the fluoroalumino silicate glass powder has a formulation containing 10 to 21% by weight of Al³⁺, 9 to 24% by weight of Si⁴⁺, 1 to 20% by weight of F⁻, and 10 to 34% by weight in total of Sr²⁺ and/or Ca²⁺.

The dental paste glass ionomer cement composition according to the present invention having the aforementioned constitution maintains the excellent characteristics of a conventional dental glass ionomer cement, i.e., considerably good affinity to a living body, excellent esthetic property with a translucent hardened body, excellent adhesion property to tooth substance, e.g., enamel and dentin, and cariostatic function of fluorine contained in fluoroalumino silicate glass powder as glass powder for the glass ionomer cement. Furthermore, the dental paste glass ionomer cement composition according to the present invention is excellent in operationality since the dental paste glass ionomer cement composition is in a two-paste system and can be immediately used by extruding the two pastes from an extruder having a mixer function, which is unlike the conventional powder-liquid type composition, in which they are weighed to prescribed amounts and mixed and kneaded on kneading paper immediately before use. Moreover, the silica aqueous sol containing colloidal silica having an average particle diameter of 1 to 100 nm dispersed in a monodisperse state in water is mixed in the prescribed amount in terms of colloidal silica amount, whereby minute pores and defects inside a hardened body of the dental glass ionomer cement and flaws on the surface of the hardened body are filled with the colloidal silica in a monodisperse state to improve the physical strength, such as the compression strength, of the hardened body.

The dental paste glass ionomer cement composition according to the present invention contains:
a first paste containing:
   20 to 60% byweight of an α-β unsaturated carboxylic acid polymer,
   10 to 60% by weight of a filler that is not reacted with the α-β unsaturated carboxylic acid polymer and is not in a monodisperse state in water,
   0.1 to 10% by weight in terms of colloidal silica amount of a silica aqueous sol containing colloidal silica having an average particle diameter of 1 to 100 nm dispersed in a monodisperse state in water to an SiO₂ concentration of 1 to 50% by weight, and
   20 to 60% by weight as the balance of water,
   and
a second paste that is reacted with the first paste by mixing therewith at a prescribed mixing ratio, the second paste containing 50 to 85% by weight of fluoroalumino silicate glass powder.

The second paste may contain as a first embodiment :
50 to 85% by weight of fluoroalumino silicate glass powder,
0.01 to 10% by weight of a thickening agent comprising a water soluble polymer material, and
20 to 45% by weight as the balance of water.

The second paste may contain as a second embodiment:
50 to 85% by weight of fluoroalumino silicate glass powder,
0.01 to 10% by weight of a thickening agent comprising a water soluble polymer material,
0.1 to 10% by weight in terms of colloidal silica amount of a silica aqueous sol containing colloidal silica having an average particle diameter of 1 to 100 nm dispersed in a monodisperse state in water to an SiO₂ concentration of 1 to 50% by weight, and
20 to 45% by weight as the balance of water.

The second paste may contain as a third embodiment :
50 to 85% by weight of fluoroalumino silicate glass powder, and
15 to 20% by weight of a polymerizable monomer having no acid group, and
in the third embodiment, at least one of the second paste and the first paste comprises a polymerization catalyst in an amount of 0.05 to 10 parts by weight per 100 parts by weight of a mixture containing the first paste and the second paste in a prescribed mixing ratio.

The α-β unsaturated carboxylic acid polymer in the first paste is a polymer of an α-β unsaturated monocarboxylic acid or an α-β unsaturated dicarboxylic acid, and is preferably a copolymer or a homopolymer containing at least one selected from acrylic acid, methacrylic acid, 2-chloroacrylic acid, 3-chloroacrylic acid, aconitic acid, mesaconic acid, maleic acid, itaconic acid, fumaric acid, glutaconic acid and citraconic acid, containing no polymerizable ethylenic unsaturated double bond, and having a weight average molecular weight of 5,000 to 40,000. In the case where the weight average molecular weight is less than 5,000, there is such a tendency that the strength of the hardened body is lowered, and the adhesion force to tooth substance is also lowered. In the case where it exceeds 40,000, there is such a tendency that the viscosity when kneading is excessively high and hard to be kneaded.

The mixing amount of the α-β unsaturated carboxylic acid polymer in the first paste is 20 to 60% by weight. In the case where the amount is less than 20% by weight, there is such a tendency that the adhesion property to tooth substance, which is a characteristic feature of a dental glass ionomer cement, is lowered, and in the case where it exceeds 60% by weight, there is such a tendency that the solubility of the hardened body is increased to impair the durability.

Specific examples of the filler in the first paste that is not reacted with the α-β unsaturated carboxylic acid polymer and is not in a monodisperse state in water include SiO₂ powder, such as colloidal silica, a mineral, such as feldspar, quartzite, quartz, kaolin and talc, crystalline glass that does not release metallic ions, such as strontium glass, barium glass and borosilicate glass, calcium carbonate, calcium phosphate, Al₂O₃ powder, TiO₂ powder, and barium sulfate. A complex filler obtained by pulverizing a polymer containing the filler may also be used. Two or more kinds of them may be used in combination. At least one selected from SiO₂, Al₂O₃ and TiO₂ is preferably used among these, and it is also preferred that the surface thereof is treated with 0.01 to 20 parts by weight of an organic compound containing a polymerizable ethylenic unsaturated double bond per 100 parts by weight of the powder component. In the case where the treatment has been effected, the final strength of the hardened body can be improved, which is useful for stability in an oral cavity. Examples of the unsaturated organic compound containing a polymerizable ethylenic double bond include a vinyl silane coupling agent, such as vinyltrimethoxysilane, vinyltriethoxysilane, γ-methacryloxypropyltrimethoxysilane, γ-methacryloxypropylmethyldimethoxysilane, vinyltrichlorosilane and vinyltris (2-methoxyethoxy) silane, and an unsaturated carboxylic acid, such as methacrylic acid, acrylic acid and maleic acid.

The filler that is not reacted with the α-β unsaturated carboxylic acid polymer and is not in a monodisperse state in water preferably has an average particle diameter of 0.02 to 10 µm. In the case where the average particle diameter exceeds 10 µm, the surface smoothness cannot be obtained to provide such a tendency that the contact feeling in an oral cavity is deteriorated. In the case where fine powder having an average particle diameter of less than 0.02 µm is used, an absolute amount of the powder is hard to be mixed, which brings about such a possibility that the hardened body is deteriorated in physical property.

The mixing amount of the filler that is not reacted with the α-β unsaturated carboxylic acid polymer and is not in a monodisperse state in water is 10 to 60% by weight. In the case where the mixing amount thereof is less than 10% by weight, there is such a tendency that the effect of improving the physical property cannot be obtained, and in the case where it exceeds 60% by weight, there is such a tendency that not only the first paste becomes stiff to make kneading with the second paste difficult, but also the physical property is rather deteriorated.

Examples of the silica aqueous sol containing colloidal silica having an average particle diameter of 1 to 100 nm dispersed in a monodisperse state in water to an SiO₂ concentration of 1 to 50% by weight include a silica aqueous sol disclosed in JP-A-4-97929 "Fine Particle Silica Aqueous Dispersion for mixing with Cement" and available under the trade name "Snowtex" from Nissan Chemical Industries, Ltd. In the case where the average particle diameter of the colloidal silica in the silica aqueous sol is less than 1 nm or exceeds 100 nm, it is not preferred since the function of improving the compression strength of the hardened body is lowered. The average particle diameter of the colloidal silica is more preferably 5 to 25 nm.

The mixing amount of the silica aqueous sol containing colloidal silica having an average particle diameter of 1 to 100 nm dispersed in a monodisperse state in water to an SiO₂ concentration of 1 to 50% by weight is 0.1 to 10% by weight in terms of colloidal silica amount.

This is because the function of improving the compression strength of the hardened body is lowered when the silica aqueous sol is mixed in an amount of less than 0.1% by weight or more than 10% by weight.

Water in the first paste is an essential component in the present invention. This is because the neutralization reaction between the fluoroalumino silicate glass and the α-β unsaturated carboxylic acid polymer proceeds in the presence of water. Furthermore, the dental paste glass ionomer cement composition of the present invention is adhered to the tooth surface in the presence of water. Accordingly, it is necessary that water is present in the dental paste glass ionomer cement composition of the present invention, and the water may be water contained in the silica aqueous sol while water may be separately added to the first paste. The mixing amount of water in the first paste is 20 to 60% by weight.

The fluoroalumino silicate glass powder in the second paste is such a substance that undergoes a neutralization reaction with the α-β unsaturated carboxylic acid polymer in the presence of water, and glass powder having been ordinarily used in a conventional dental glass ionomer cement can be used with no particular limitation. Among these, such a fluoroalumino silicate glass powder is preferred that has an average particle diameter of 0.02 to 10 µm and a specific gravity of 2.4 to 4.0, and contains as major component Al³⁺, Si⁴⁺, F⁻, O²⁻, and Sr²⁺ and/or Ca²⁺. In the case where the average particle diameter exceeds 10 µm, there is such a tendency that the surface smoothness on the surface of the hardened body of the dental glass ionomer cement is lost to deteriorate the contact feeling in an oral cavity, and in the case where it is less than 0.02 µm, an absolute amount of the powder is hard to be mixed, which brings about such a possibility that the hardened body is deteriorated in physical property. The particle diameter may be measured by an ordinary method and expressed by an average value of the major diameter and the minor diameter. The fluoroalumino silicate glass powder preferably has a formulation containing 10 to 21% by weight of Al³⁺, 9 to 24% by weight of Si⁴⁺, 1 to 20% by weight of F⁻, and 10 to 34% by weight in total of Sr²⁺ and/or Ca²⁺. The proportion of the major components exerts great influence on the operationality and physical property of the dental glass ionomer cement, such as the hardening speed, the final strength and the solubility. In the case where the proportion of Al³⁺ is less than 10% by weight, there is such a tendency that the hardening of the dental glass ionomer cement is reduced, and the strength thereof is lowered, and in the case where it exceeds 21% by weight, there is such a tendency that the glass is difficultly produced, and the transparency thereof is lowered to deteriorate aesthetic property. In the case where the proportion of Si⁴⁺ is less than 9% by weight, the glass is difficultly produced, and in the case where it exceeds 24% by weight, there is such a tendency that the hardening speed of the dental glass ionomer cement is reduced, and the strength thereof is lowered to provide a problem in durability. In the case where the proportion of F⁻ is less than 1% by weight, the operation margin when kneading the dental glass ionomer cement composition is reduced to make the operation of use difficult, and in the case where it exceeds 20% by weight, the final hardening time of the dental glass ionomer cement is prolonged, and the solubility in water thereof is increased to impair the durability. In the case where the total proportion of Sr²⁺ and/or Ca²⁺ is less than 10% by weight, there is such a tendency that the hardening reaction of the dental glass ionomer cement cannot be sharpened to prolong the hardening time, and further the glass is difficultly produced, and in the case where it exceeds 34% by weight, there is such a tendency that the operation margin time of the dental glass ionomer cement is shortened to make practical use difficult due to the excessively high hardening speed, and the solubility in water is increased to impair the durability.

The fluoroalumino silicate glass powder can be obtained by pulverizing fluoroalumino silicate glass, which has been produced in an ordinary glass production method, in a ball mill or the like, and then sieving it to obtain powder having an average particle diameter within the desired range.

The fluoroalumino silicate glass powder is mixed in the second paste in an amount of 50 to 85% by weight. In the case where the mixing amount is less than 50% by weight, there is such a tendency that the hardened body of the dental glass ionomer cement is deteriorated in physical property, and in the case where it exceeds 85% by weight, it is not preferred since there is such a tendency that the second paste becomes stiff to make the operationality in mixing with the first paste deteriorated.

A thickening agent comprising a water soluble polymer material for imparting a suitable viscosity to the paste is mixed in the second paste in the case where the dental paste glass ionomer cement composition of the present invention is an ordinary glass ionomer cement composition containing no polymerizable monomer.

The thickening agent may be freely selected as far as it has no toxicity to a human body. Examples thereof include calcium carboxymethyl cellulose, sodium carboxymethyl cellulose, starch, sodium starch glycolate, sodium starch phosphate, methyl cellulose, sodium polyacrylate, alginic acid, sodium alginate, propylene glycol alginate ester, casein, sodium casein, polyethylene glycol, ethyl cellulose, hydroxyethyl cellulose, gluten, locust bean gum and gelatin, and among these, calcium carboxymethyl cellulose and sodium carboxymethyl cellulose are preferred since they are inexpensive and provide a high viscosity increasing effect with a small amount. Two or more kinds of the thickening agent comprising a water soluble polymer material may be used in combination.

The thickening agent comprising a water soluble polymer material is mixed in the second paste in an amount of 0.01 to 10% by weight. In the case where the mixing amount is less than 0.01% by weight, there is such a tendency that the viscosity increasing effect with the thickening agent cannot be obtained, and in the case where it exceeds 10% by weight, there is such a tendency that the hardened body is deteriorated in physical property. The mixing amount of the thickening agent comprising a water soluble polymer material is preferably as small as possible for preventing the physical property of the hardened body from being deteriorated, and is more preferably 0.02 to 4% by weight.

In the case where the thickening agent comprising a water soluble polymer material is mixed in the second paste, it is necessary that 20 to 45% by weight as the balance of water is mixed in the second paste for exhibiting the desired viscosity through dissolution of the thickening agent.

In the case where the second paste contains the fluoroalumino silicate glass powder, the thickening agent comprising a water soluble polymer material, and water, it is preferred that 0.1 to 10% by weight in terms of colloidal silica amount of a silica aqueous sol containing colloidal silica having an average particle diameter of 1 to 100 nm dispersed in a monodisperse state in water to an SiO₂ concentration of 1 to 50% by weight is mixed as similar to the first paste, since the effect of improving the compression strength of the hardened body is further enhanced. The mixing amount thereof is necessarily 0.1 to 10% byweight because of the same grounds as in the silica aqueous sol mixed in the first paste.

In the case where the dental paste glass ionomer cement composition of the present invention is a resin-reinforced glass ionomer cement composition containing a polymerizable monomer, a polymerizable monomer having no acid group is mixed in the second paste for improving the physical strength of the hardened body, particularly the flexural strength thereof. The polymerizable monomer having no acid group is a polymerizable unsaturated organic compound having at least one of a CH₂=CR-COO- group (wherein R represents H or CH₃), and designates a polymerizable unsaturated organic compound having an acryloid group or a methacryloid group, such as esters of acrylic acid and methacrylic acid. It is necessary that the polymerizable monomer is not reacted with the fluoroalumino silicate glass powder contained in the second paste. In other words, the polymerizable monomer does not contain an acid group capable of being reacted with the fluoroalumino silicate glass, such as an acid group containing carboxylic acid, phosphoric acid, sulfur or boric acid. The polymerizable monomer having no acid group is not limited to the aforementioned examples, and preferably does not contain such an acid group that undergoes an acid-base reaction with the fluoroalumino silicate glass powder.

Preferred examples of the polymerizable monomer having no acid group include hydroxy methacrylate, glycidyl methacrylate, triethylene glycol dimethacrylate, neopentyl glycol dimethacrylate, 1,6-hexanediol dimethacrylate, 1,3-butanediol dimethacrylate, 2-hydroxy-1-acryloxy-3-methacryloxypropane, hexamethylene dicarbamate and di-2-methacryloxyethyl-2,2,4-triethylhexamethylene dicarbamate, plural kinds of which may be used in combination.

The mixing amount of the polymerizable monomer having no acid group is 15 to 50% by weight. In the case where the mixing amount thereof is less than 15% by weight, there are cases where the initial hardening property and the physical property of the dental glass ionomer cement are deteriorated, and in the case where it exceeds 50% by weight, there is such a tendency that the adhesion property to tooth substance, which is a characteristic feature of a dental glass ionomer cement, is lowered.

In the case where the second paste contains the fluoroalumino silicate glass powder and the polymerizable monomer containing no acid group, at least one of the second paste and the first paste comprises a polymerization catalyst in an amount of 0.05 to 10 parts by weight per 100 parts by weight of the mixture containing the first paste and the second paste in a prescribed mixing ratio.

The present invention includes two cases where the polymerization catalyst used is a chemical polymerization catalyst and is a photopolymerization catalyst.

Examples of the chemical polymerization catalyst include an organic aromatic compound having at least one of an -SO₂- group, such as an aromatic sulfinic acid, an alkali salt thereof, and an aromatic sulfonyl compound. Specific examples thereof include sodium p-toluenesulfinate, lithium p-toluenesulfinate, benzenesulfinic acid, sodium benzenesulfinate, p-toluenesulfonyl chloride, P-toluenesulfonyl fluoride, O-toluenesulfonyl isocyanate, sodium p-acetamidebenzenesulfinate, and among these, sodium p-toluenesulfinate and sodium benzenesulfinate are preferred. The organic aromatic compound having at least one of an -SO₂- group may be a hydrate salt thereof. When the chemical polymerization catalyst is mixed simultaneously with the polymerizable monomer having no acid group, the catalyst exerts a function of hardening the polymerizable monomer having no acid group. Accordingly, the chemical polymerization catalyst is mixed in the first paste. The chemical polymerizationcatalystmay bemicro-encapsulated with a polymer compound, such as cellulose, to ensure the stability thereof for a long period of time. The micro-encapsulation of the chemical polymerization catalyst enables that the chemical polymerization catalyst is mixed not only in the first paste but also in the second paste through appropriate selection of the polymer compound. It is preferred in the present invention that a water insoluble polymer compound is used for micro-encapsulation of the chemical polymerization catalyst, which is mixed in the second paste. This is because a water insoluble polymer compound has higher storage stability than a water soluble polymer compound in general.

Examples of the polymer compound used for micro-encapsulation of the chemical polymerization catalyst include a water insoluble polymer compound, such as ethyl cellulose, cellulose acetate, polyvinyl formal, cellulose acetate phthalate, cellulose acetate butyrate, hydroxypropylmethyl cellulose phthalate and EUDRAGIT, a trade name, produced by Rohm & Hass GmbH, and a water soluble polymer compound, such as polyvinyl alcohol, carboxymethyl cellulose, methyl cellulose and hydroxyethyl cellulose. The average particle diameter of the micro-encapsulated chemical polymerization catalyst is preferably 0.1 to 30 µm for dispersing in the paste and for attaining as much collapsing property as possible. In the case where the average particle diameter thereof is less than 0.1 µm, the micro-encapsulation becomes difficult, and in the case where it exceeds 30 µm, there is such a tendency that the dispersibility in the paste is deteriorated.

In applications where the dental paste glass ionomer cement composition can be sufficiently irradiated with light, such as restoration of a caries cavity, the chemical polymerization catalyst may be replaced by a photopolymerization catalyst to polymerize the polymerizable monomer having no acid group with visible light, whereby hardening of the paste dental cement composition can be started at an arbitrary timing desired by an operator during the period until the neutralization reaction of the fluoroalumino silicate glass powder and the α-β unsaturated carboxylic acid polymer. In this case, the photopolymerization catalyst may be generally a combination of a sensitizer and a reducing agent.

As the sensitizer, a compound capable of polymerizing the polymerizable monomer with an action of visible light having a wavelength of 390 to 830 nm may be used. Examples thereof include camphor quinone, benzyl, diacetyl, benzyldimethylketal, benzyldiethylketal, benzyldi(2-methoxyethyl)ketal, 4,4'-dimethylbenzyldimethylketal, anthraquinone, 1-chloroanthraquinone, 2-chloroanthraquinone, 1,2-benzyanthraquinone, 1-hydroxyanthraquinone, 1-methylanthraquinone, 2-ethylanthraquinone, 1-bromoanthraquinone, thioxanthone, 2-isopropylthioxanthone, 2-nitrothioxanthone, 2-methylthioxanthone, 2,4-dimethylthioxanthone, 2,4-diethylthioxanthone, 2,4-diisopropylthioxanthone, 2-chloro-7-trifluoromethylthioxanthone, thioxanthone-10,10-dioxide, thioxanthone-10-oxide, benzoin methyl ether, benzoin ethyl ether, isopropyl ether, benzoin isobutyl ether, benzophenone, bis(4-dimethylaminophenyl) ketone, 4,4'-bisdiethylaminobenzophenone, an acylphosphine oxide, such as (2,4,6-trimethylbenzoyl)diphenylphosphine oxide, and a compound containing an azide group, which may be used solely or in combination of plural kinds thereof.

As the reducing agent, a tertiary amine is generally used. Preferred examples of the tertiary amine include N,N-dimethyl-p-toluidine, N,N-dimethylaminoethyl methacrylate, triethanolamine, methyl 4-dimethylaminobenzoate, ethyl 4-dimethylaminobenzoate and isoamyl 4-dimethylaminobenzoate. Other examples of the reducing agent include a sodium sulfinate derivative and an organic metallic compound. These reducing agents may be used solely or in combination of plural kinds thereof.

The combination of the sensitizer and the reducing agent generally has no function of hardening the polymerizable monomer having no acid group under no irradiation with light, and the sensitizer and the reducing agent are not reacted with each other. Therefore, the sensitizer and the reducing agent may be mixed with either the first or second paste, and they may be mixed separately or simultaneously. As similar to the chemical polymerization catalyst, the photopolymerization catalyst having a function of directly polymerizing the polymerizable monomer having no acid group is mixed in the first paste, and can be mixed not only in the first paste but also in the second paste, when the photopolymerization catalyst is micro-encapsulated through appropriate selection of the polymer compound.

As the α-β unsaturated carboxylic acid polymer used in the first paste of the dental paste glass ionomer cement composition, the following carboxylic acid polymers were prepared.

### Carboxylic Acid Polymer 1

An acrylic acid-maleic acid copolymer having a weight average molecular weight of 18, 000 was prepared.

### Carboxylic Acid Polymer 2

Polymaleic acid having a weight average molecular weight of 16,000 and polyacrylic acid having a weight average molecular weight of 30, 000 were mixed at a weight ratio of 5/1 to prepare a carboxylic acid polymer.

### Carboxylic Acid Polymer 3

Polymaleic acid having a weight average molecular weight of 16,000 and polyacrylic acid having a weight average molecular weight of 24 , 000 were mixed at a weight ratio of 5/1 to prepare a carboxylic acid polymer.

As the filler not reacted with the α-β unsaturated carboxylic acid polymer and not in a monodisperse state in water used in the first paste of the dental paste glass ionomer cement composition, the following fillers were prepared.

### Fine Powder Quartz

Fine powder quartz having an average particle diameter of 4 µm (Crystallite VX-S, a trade name, produced by Tatsumori Co., Ltd.) was prepared.

### Fine Powder Aluminum Oxide

Fine powder aluminum oxide having an average particle diameter of 5 µm (Admafine Alumina A-500, a trade name, produced by Admatechs Co., Ltd.) was prepared.

### Fine Powder Titanium Oxide

Fine powder titanium oxide having an average particle diameter of 0.4 µm (KRONOS KA-50, a trade name, produced by Titanium Kogyo Co., Ltd.) was prepared.

### Treated Fine Powder Quartz 1

20 g of a 10% ethyl alcohol solution of γ-methacryloxypropyltrimethoxysilane was added to 100 g of the aforementioned fine powder quartz, and after sufficiently agitating in a mortar, the mixture was dried at 110°C for 2 hours by using a dryer to prepare a silane-treated fine powder quartz.

### Treated Fine Powder Quartz 2

20 g of a 10% ethyl alcohol solution of vinylethoxysilane was added to 100 g of the aforementioned fine powder quartz, and after sufficiently agitating in a mortar, the mixture was dried at 110°C for 2 hours by using a dryer to prepare a silane-treated fine powder quartz.

As the silica aqueous sol used in the first paste, or both the first paste and the second paste of the dental paste glass ionomer cement composition, the following sols were prepared.

### Sol 1

A sol containing colloidal silica having an average particle diameter of 8 to 11 nm dispersed in a monodisperse state in water to an SiO₂ concentration of 20% by weight (Snowtex OS, a trade name, produced by Nissan Chemical Industries, Ltd.) was prepared.

### Sol 2

A sol containing colloidal silica having an average particle diameter of 10 to 20 nm dispersed in a monodisperse state in water to an SiO₂ concentration of 20% by weight (Snowtex O, a trade name, produced by Nissan Chemical Industries, Ltd.) was prepared.

As the fluoroalumino silicate glass powder used in the second paste of the dental paste glass ionomer cement composition, the following kinds of glass powder were prepared.

### Glass Powder A

22 g of aluminum oxide, 43 g of silicic anhydride, 12 g of calcium fluoride, 15 g of calcium phosphate and 8 g of strontium carbonate were sufficiently mixed and maintained in a high temperature electric furnace at 1,200°C for 5 hours to melt the components. After melting, the mixture was cooled and pulverized by using a ball mill for 10 hours, followed by sieving with a 200-mesh (ASTM) sieve, to obtain glass powder.

### Glass Powder B

23 g of aluminum oxide, 41 g of silicic anhydride, 10 g of calcium fluoride, 13 g of calcium phosphate and 13 g of aluminum phosphate were sufficiently mixed and maintained in a high temperature electric furnace at 1, 100°C for 5 hours to melt the components. After melting, the mixture was cooled and pulverized by using a ball mill for 10 hours, followed by sieving with a 200-mesh (ASTM) sieve, to obtain glass powder. 20 g of a 10% ethyl alcohol solution of vinyltriethoxysilane was added to 100 g of the glass powder, and after sufficiently agitating in a mortar, the mixture was dried at 110°C for 2 hours by using a dryer to prepare silane-treated fluoroalumino silicate glass powder.

As the thickening agent comprising a water soluble polymer material used in the second paste of the dental paste glass ionomer cement composition, the following thickening agents were prepared.

### Thickening Agent 1

Sodium carboxymethyl cellulose (CellogenHF-600F, a trade name, produced by Dai-ichi Kogyo Seiyaku Co., Ltd.) was prepared.

### Thickening Agent 2

Hydroxyethyl cellulose (Cellogen F-3H, a trade name, produced by Dai-ichi Kogyo Seiyaku Co., Ltd.) was prepared.

As the polymerizable monomer having no acid group used in the second paste of the dental paste glass ionomer cement composition, the following monomer liquids were prepared.

### Monomer Liquid 1

Hydroxyethyl methacrylate, 2-hydroxy-1-acryloxy-3-methacryloxypropane, di-2-methacryloxyethyl-2,2,4-triethylhexamethylene dicarbamate and glycidyl methacrylate were mixed at a weight ratio of 15/4/4/4 to prepare a monomer liquid.

### Monomer Liquid 2

Hydroxyethyl methacrylate, 2-hydroxy-1-acryloxy-3-methacryloxypropane, di-2-methacryloxyethyl-2,2,4-triethylhexamethylene dicarbamate and 1,6-hexanediol methacrylate were mixed at a weight ratio of 10/3/4/3 to prepare a monomer liquid.

As the polymerization catalyst used in the first paste and/or the second paste of the dental paste glass ionomer cement composition, the following polymerization catalysts were prepared.

### Polymerization Catalyst 1

Sodium benzenesulfinate was prepared.

### Polymerization Catalyst 2

p-toluenesulfonyl chloride was prepared.

### Polymerization Catalyst 3

5 g of hydroxypropylmethyl cellulose phthalate was dissolved in 500 mL of methylene chloride, in which 10 g of sodium benzenesulfinate adjusted to have an average particle diameter of 10 µm was then dispersed. The dispersion was dried under stirring and cooling to prepare a micro-encapsulated polymerization catalyst having an average particle diameter of 13 µm, which was used as a core material. 10 g of the core material was added to 500 mL of distilled water having 10 g of polyvinyl alcohol dissolved therein, and the mixture was dried under stirring and cooling to prepare a micro-encapsulated polymerization catalyst having an average particle diameter of 17 µm.

The aforementioned components were mixed at the proportions shown in Table 1 below to produce the first and second pastes, which were then mixed at the weight mixing ratios shown in Table 1 to prepare dental paste glass ionomer cement compositions. The dental paste glass ionomer cement compositions were measured for compression strength ratio in the following manner. The results obtained are shown in Table 1.

The first paste and the second paste was mixed and kneaded at the weight mixing ratio shown in Table 1, and the mixture was charged in a cavity having an inner diameter of 4 mm and a length of 6 mm of a metallic mold and pressed by a glass plate through a cellophane sheet to obtain a hardened body in a cylindrical shape. The resulting hardened body was immersed in distilled water at 37°C for 24 hours and then subjected to a compression strength test by using a multipurpose tester (Autograph, a trade name, produced by Shimadzu Corp.) at a crosshead speed of 1 mm/min. The compression strength ratios of the dental paste glass ionomer cement compositions were evaluated with the following standard. In the case where the weight mixing ratio of the first paste and the second paste was 1/1, the compression strength (97 MPa) of Comparative Example 1 containing no silica aqueous sol was designated as 100. In the case where the weight mixing ratio of the first paste and the second paste was 1/3, the compression strength (131 MPa) of Comparative Example 2 containing no silica aqueous sol was designated as 100. In the case where the weight mixing ratio of the first paste and the second paste was 1/5, the compression strength (157 MPa) of Comparative Example 3 containing no silica aqueous sol was designated as 100.

As a result, it was confirmed that the compression strengths of the dental paste glass ionomer cement compositions of the present invention were improved by 10 to 20% as compared to the comparative dental paste glass ionomer cement compositions containing no silica aqueous sol in all the cases where the weight mixing ratio of the first paste and the second paste was 1/1, 1/3 and 1/5.

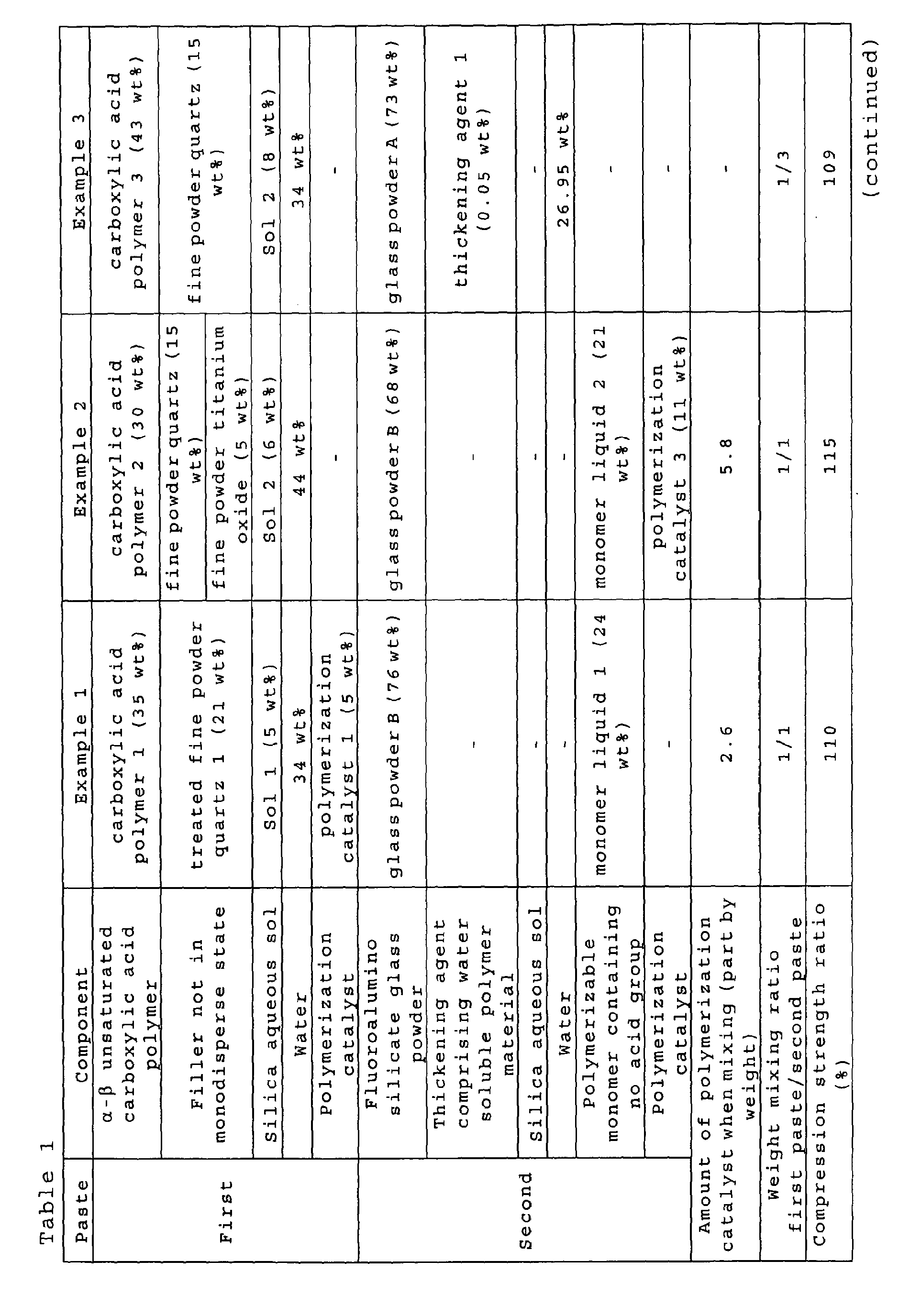

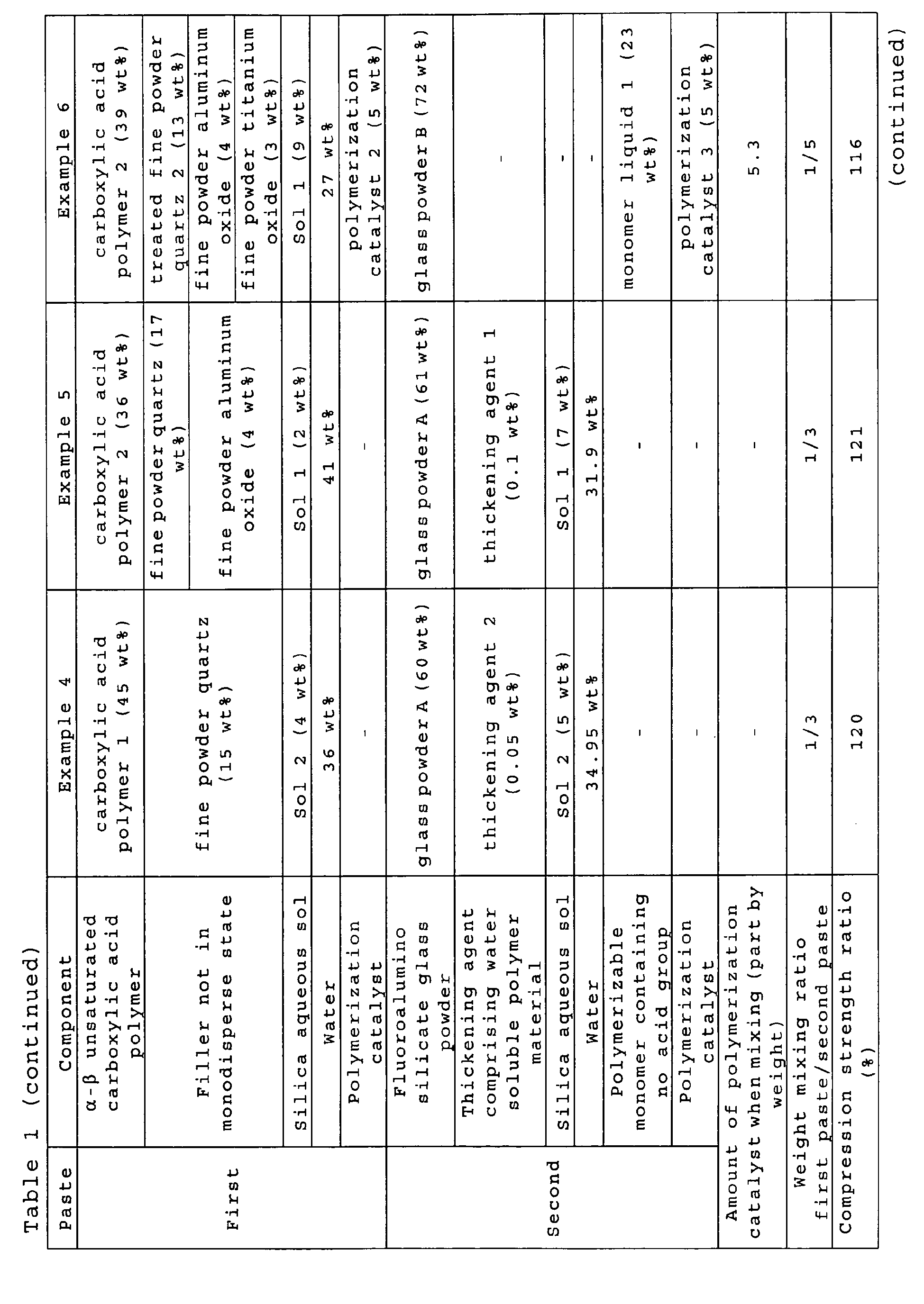

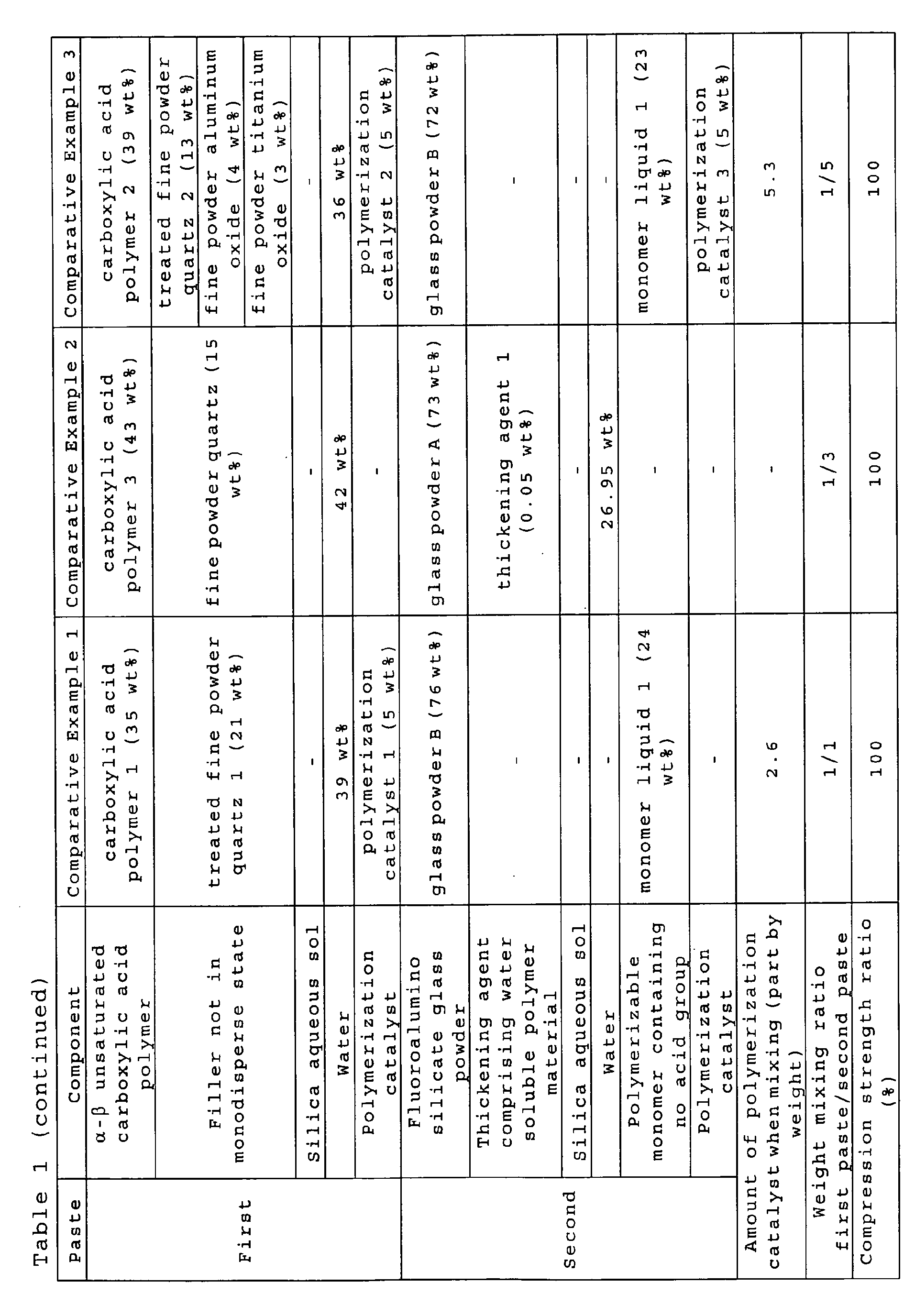

## Claims

1. A dental paste glass ionomer cement composition comprising:
a first paste comprising:
20 to 60% by weight of an α-β unsaturated carboxylic acid polymer,
10 to 60% by weight of a filler that is not reacted with the α-β unsaturated carboxylic acid polymer and is not in a monodisperse state in water,
0.1 to 10% by weight in terms of colloidal silica amount of a silica aqueous sol containing colloidal silica having an average particle diameter of 1 to 100 nm dispersed in a monodisperse state in water to an SiO₂ concentration of 1 to 50% by weight, and
20 to 60% by weight as the balance of water,
and
a second paste that is reacted with the first paste by mixing therewith at a prescribed mixing ratio,
the second paste comprising:
50 to 85% by weight of fluoroalumino silicate glass powder,
0.01 to 10% by weight of a thickening agent comprising a water soluble polymer material, and
20 to 45% by weight as the balance of water.

2. The dental paste glass ionomer cement composition as claimed in claim 1, wherein the second paste comprises:
50 to 85% by weight of fluoroalumino silicate glass powder,
0.01 to 10% by weight of a thickening agent comprising a water soluble polymer material,
0.1 to 10% by weight in terms of colloidal silica amount of a silica aqueous sol containing colloidal silica having an average particle diameter of 1 to 100 nm dispersed in a monodisperse state in water to an SiO₂ concentration of 1 to 50% by weight, and
20 to 45% by weight as the balance of water.

3. The dental paste glass ionomer cement composition as claimed in claim 1, wherein the second paste comprises:
50 to 85% by weight of fluoroalumino silicate glass powder, and
15 to 50% by weight of a polymerizable monomer having no acid group, and
at least one of the second paste and the first paste comprises a polymerization catalyst in an amount of 0.05 to 10 parts by weight per 100 parts by weight of a mixture containing the first paste and the second paste in a prescribed mixing ratio.

4. The dental paste glass ionomer cement composition as claimed in one of claims 1 to 3, wherein the α-β unsaturated carboxylic acid polymer is a copolymer or a homopolymer containing at least one selected from acrylic acid, methacrylic acid, 2-chloroacrylic acid, 3-chloroacrylic acid, aconitic acid, mesaconic acid, maleic acid, itaconic acid, fumaric acid, glutaconic acid and citraconic acid, containing no polymerizable ethylenic unsaturated double bond, and having a weight average molecular weight of 5,000 to 40,000.

5. The dental paste glass ionomer cement composition as claimed in one of claims 1 to 4, wherein the filler that is not reacted with the α-β unsaturated carboxylic acid polymer and is not in a monodisperse state in water is at least one selected from SiO₂, Al₂O₃ and TiO₂.

6. The dental paste glass ionomer cement composition as claimed in one of claims 1 to 5, wherein the fluoroalumino silicate glass powder has a formulation comprising 10 to 21% by weight of Al³⁺, 9 to 24% by weight of Si⁴⁺, 1 to 20% by weight of F⁻, and 10 to 34% by weight in total of Sr²⁺ and/or Ca²⁺.
